# EUROPEAN PATENT APPLICATION

(11) **EP 4 521 112 A1**
(43) Date of publication of application: **12.03.2025**
(21) Application number: 23205852.9
(22) Date of filing: 25.10.2023
(51) Int. Cl.: G01N 33/53, C12Q 1/6804, C12Q 1/6823

(54) **MARKER, KIT AND METHOD FOR ANALYSING A BIOLOGICAL SAMPLE**

(30) Priority: 06.09.2023 EP 23195848
(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

A marker for analysing a biological sample is provided. The marker comprising an affinity reagent with a backbone, and a barcode oligonucleotide attached to the backbone. The backbone is configured to specifically bind to a target analyte. The marker further comprises a label with a label oligonucleotide and at least one labelling moiety. The label oligonucleotide and the barcode oligonucleotide of the affinity reagent are configured to hybridise to each other. One of the label oligonucleotide and the barcode oligonucleotide is sensitive to a degradation agent and the other one of the label oligonucleotide and the barcode oligonucleotide is resistant to the degradation agent. In further aspects a kit and a method for analysing the biological sample with the marker are provided.

## Description

### Technical field

The invention relates to a marker for analysing a biological sample comprising an affinity reagent and a label. In a further aspect, a method for analysing a biological sample with the marker is provided.

### Background

Affinity reagents comprise a chemically heterogeneous group and include for example antibodies, nucleic acid probes, and aptamers. Aptamers are typically short nucleic acid sequences that fold into 3-dimensional structures, which are able to bind a target analyte with high affinity and specificity. The use of barcoded affinity reagents such as antibodies, nanobodies, aptamers, or *in situ* probes, which are connected to an oligonucleotide sequence that is unique to said affinity reagent and may contain one or more predetermined sequences that can be hybridised by detectable labels that comprise a complementary barcode, is an effective strategy for cyclical labelling. Such cyclical labelling is used when biological samples shall be analysed for the presence of a high number of target analytes.

Fluorescence microscopy allows for imaging a biological sample with high spatial resolution but involves only a low number of different fluorescent dyes, typically between 1 and 5. The available markers comprising the dyes have to include markers that are used to identify cell types, functional markers like protein-of-interest, and general morphological markers in the same experiment. This means that cell types in most imaging experiments are merely poorly identified. This also means that rather broad multi cell type populations are being studied, which severely limits the predictive power and translational value of the results generated. While modern approaches that allow for a much more reliable and robust identification of cell types, e.g. based on the analysis of genetic regulatory networks (GRNs), exist, they require a much higher number of markers with distinguishable dyes to be readout from the sample.

While in the adjacent field of cytometry, mass cytometry and imaging mass cytometry techniques can distinguish between around 12 to 30 different markers, they do so with a low spatial resolution.

Spatial profiling techniques based on RNA capture can distinguish a number of different markers several orders of magnitude higher, albeit at an even lower spatial resolution as they are based on hybridizing oligonucleotides to the sample and then selectively releasing bound oligonucleotides in a region-selective fashion followed by next-generation sequencing of the released oligonucleotides.

### Summary

It is an object to provide a barcoded marker that enables efficient repeated staining of a biological sample and a method for analysing a biological sample with the marker.

The aforementioned object is achieved by the subject-matter of the independent claims. Advantageous embodiments are defined in the dependent claims and the following description.

In one aspect, a marker for analysing a biological sample is provided. The marker comprising an affinity reagent with a backbone, and a barcode oligonucleotide attached to the backbone. The backbone is configured to specifically bind to a target analyte. The marker further comprises a label with a label oligonucleotide and at least one labelling moiety. The label oligonucleotide and the barcode oligonucleotide of the affinity reagent are configured to hybridise to each other. One of the label oligonucleotide and the barcode oligonucleotide is sensitive to a degradation agent and the other one of the label oligonucleotide and the barcode oligonucleotide is resistant to the degradation agent. This enables preferably degrading the sensitive one of the label oligonucleotide and the barcode oligonucleotide and therefore removing it from the affinity reagent, in particular the affinity reagent backbone, by addition of the degradation agent. Preferably, the affinity reagent, in particular the affinity reagent backbone, remains intact in the presence of the degradation agent. In particular, the affinity reagent backbone may continue to be configured to specifically bind to the target analyte in the presence of the degradation agent. Thus, preferably, the affinity reagent backbone is resistant to the degradation agent.

The degradation agent causes degradation only of the sensitive one of the barcode oligonucleotide and the label oligonucleotide, in particular, the degradation agent may digest or fragment the sensitive oligonucleotide. This degradation generally results in loss of function of the oligonucleotide, in particular, the sensitive oligonucleotide no longer binds or is no longercapable of bindingto the resistant oligonucleotide. In other words, the sensitive oligonucleotide is configured to be (specifically) degraded by the degradation agent. Preferably, the degradation of the sensitive oligonucleotide is irreversible. Thus, the degradation agent causes the label, in particular the label oligonucleotide, to be removed from the barcode oligonucleotide and render the barcode oligonucleotide accessible to another label in another round of labelling.

The barcode oligonucleotide is preferably covalently attached to the backbone of the affinity reagent. The term backbone of the affinity reagent is not limitingto a particular part or element. For example, in case the backbone of the affinity reagent consists essentially of nucleic acids, the backbone of the affinity reagent does not exclusively refer to a phosphate backbone of the nucleic acid. Thus, the barcode oligonucleotide may be attached to any of a phosphate backbone, a pentose sugar, or a nucleobase of the nucleic acid of the backbone of the affinity reagent.

In another example, in case the backbone of the affinity reagent consists essentially of amino acids, i.e. a polypeptide, the backbone of the affinity reagent does not exclusively refer to a polypeptide backbone (i.e. peptide chain) of the polypeptide, protein, antibody, nanobody, or aptabody. Thus, the barcode oligonucleotide may be attached to any of a polypeptide, an amino acid, or an amino acid residue, or a modification of an amino acid residue for example a glycosylation, prenylation, or other post-translational modification of the backbone of the affinity reagent.

For example, in case the backbone of the affinity reagent consists essentially of a non-naturally occurring polymer the same applies. Also in this case, the backbone does not exclusively refer to the backbone of the polymer, i.e. the repeating polymeric unit, but also to the residues and any modification of residues.

The documents WO 2022/242849 A1 and WO 2022/242887 A1, the complete contents of which are incorporated herein by reference, propose markers and methods for increasing the number of distinguishable markers that can be used in a single fluorescence microscopy experiment. Each marker comprises a unique combination of dyes forming a code, that in principle identifies the respective marker. However, certain ambiguities remain, in particular when a large number of markers is in close proximity of each other. In order to resolve these ambiguities, it is necessary to remove the markers and repeat the image acquisition with a different set of markers. In particular, in the document WO 2022/242887 A1, a method is disclosed, which enables high-plex analysis using microscopy and an encoding/decoding process, that is based on cyclic imaging. A core aspect of this method is a referred to as "code swapping", which refers to the marking of an analyte A with an n-th code in the n-th round and an n+1th code in the n+1th round. The present invention provides a particularly preferred way, to enable the swapping of codes, i.e. combinatorial labels comprising multiple colors or combinations of different labeling moieties, in an efficient manner in a real biological sample.

While the present invention is very well suited to perform multiplex and high-plex analysis using microscopes or plate readers for example, it can also be used for many other applications, which may benefit from a rapid, robust, and cost-efficient way to mark analytes in a cyclical fashion.

Barcoding affinity reagents is an efficient strategy that allows various modes of cyclic imaging processes, that are referred to as marker cycling, label cycling, and barcode cycling amongst others in this document.

Preferably, the backbone of the affinity reagent consists essentially of amino acids. For example, the backbone of the affinity reagent may be a protein. This enables the affinity reagent to be resistant to oligonucleotide specific degradation agents. In particular, the protein may carry post-translational modifications. Thus, the amino acids of the backbone may be glycosylated, for example.

It is particularly preferred that the backbone of the affinity reagent is an antibody or an antibody fragment. This enables the affinity reagent to be particularly specific to a particular target analyte such as a protein.

In an alternative, the backbone of the affinity reagent preferably consists essentially of nucleic acids. This enables the affinity reagent to specifically bind to a target analyte such as an mRNA, for example. Preferably, the nucleic acid of the affinity reagent backbone is resistant to the degradation agent.

Preferably, the backbone of the affinity reagent is configured to specifically bind to the target analyte by hybridising of a continuous sequence of nucleotides to a complementary continuous target sequence of nucleotides. This enables the affinity reagent to specifically bind to a target analyte such as an mRNA, for example, similarly to a FISH-probe. In this case, the backbone is configured to specifically bind to the target analyte by hybridising of a continuous sequence of nucleotides to a complementary continuous target sequence of nucleotides.

Preferably, the backbone of the affinity reagent has a complex structure and the backbone is configured to specifically bind to the target analyte by its complex structure. The complex structure may preferably be a secondary, tertiary, or quaternary structure of the backbone. This enables the affinity reagent to specifically bind to a target analyte such as protein, for example. In the case the backbone has a complex structure, discontinuous nucleotides or stretches of the nucleic acid sequence interact with and/or specifically bind to the target analyte.

It is particularly preferred that the backbone of the affinity reagent is an aptamer. This enables efficiently generating backbones of the affinity reagent that are specific to a range of target analytes, such as various proteins. Aptamers maybe generated in various ways, with SELEX being the most typical route. While most aptamers comprise nucleic acid sequences (RNA, DNA, XNA), they may also be generated by other polymers such as for example peptides. Moreover, recently aptabodies have been proposed, which are essentially aptamers with a nucleic acid backbone, comprising modifications, which are naturally found on proteins, for example glycosylation.

Preferably, one of the label oligonucleotide and the barcode oligonucleotide consists essentially of a nucleic acid sensitive to a degradation agent, for example, a natural nucleic acid, and the other one of the label oligonucleotide and the barcode oligonucleotide consists essentially of a nucleic acid analogue resistant to the degradation agent. This enables efficiently generating the label and barcode oligonucleotides with either sensitivity or resistance to the degradation agent. Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered.

The nucleic acid analogue may be an artificial nucleic acid or a xeno nucleic acid. Whereas natural nucleic acids or naturally occurring nucleic acids (DNA and RNA) are generally sensitive to degradation agents such as nucleases, nucleic acid analogues are generally resistant to degradation agents such as nucleases. Further, in case the affinity reagent backbone consists essentially of nucleic acids, these nucleic acids are preferably nucleic acid analogues resistant to the degradation agent.

Preferably, the label oligonucleotide consists essentially of a natural nucleic acid and the barcode oligonucleotide consists essentially of a nucleic acid analogue. This enables efficiently generating affinity reagents resistant to the degradation agent and labels that can be efficiently degraded and therefore selectively removed from the affinity reagent. In this case, the affinity reagent backbone preferably consists essentially of nucleic acid analogues. Thus, the barcode oligonucleotide and the backbone are preferably resistant to the degradation agent.

It is particularly preferred that the nucleic acid analogue comprises at least one of a 1,5-anhydorhexitol nucleic acid, a cyclohexene nucleic acid, a threose nucleic acid, a glycol nucleic acid, a locked nucleic acid, a peptide nucleic acid, a fluoro arabino nucleic acid, a bridged nucleic acid, a morpholino, a phosphorothioate backbone, a L-pentose backbone, a nucleic acid comprising at least one modified nucleoside. This enables high resistance to the degradation agent.

Preferably, the at least one labelling moiety is optically detectable. This enables generating markers that are easily detected, for example, by means of a microscope.

Preferably, the degradation agent is a nuclease. This enables easily degrading the sensitive barcode or label oligonucleotide.

In a particularly preferred combination, the sensitive barcode or label oligonucleotide is sensitive to a nuclease degradation agent, such as DNase I, and the resistant barcode or label oligonucleotide is resistant to the nuclease degradation agent, such as DNase I. In this case, the resistant barcode or label oligonucleotide preferably consists essentially of a nucleic acid analogue such as a non-naturally occurring nucleic acid or XNA.

Preferably, the degradation agent and the sensitive label oligonucleotide or sensitive barcode oligonucleotide are configured such that applying the degradation agent releases the barcode oligonucleotide from a label oligonucleotide-barcode oligonucleotide duplex, such that the barcode oligonucleotide is able to hybridise to another label oligonucleotide.

Preferably, the degradation agent is an artificially engineered peptidase or protease configured to degrade a peptide nucleic acid or a xeno nucleic acid.

Preferably, the degradation agent is silver, iodine, or mercury and the sensitive nucleic acid analogue is a phosphorothioate-modified nucleic acid.

Preferably, the degradation agent is based on an inorganic or organic chemistry that allows the degradation of the sensitive nucleic acid analogue.

In a further aspect, a kit is provided comprising at least one marker and the degradation agent.

Further, a kit is provided comprising at least one barcode oligonucleotide and chemical components required to conjugate said barcode oligonucleotide to an affinity reagent backbone in order to generate the marker.

Further, a kit is provided comprising the degradation agent and at least one label comprising a label oligonucleotide in order to generate the marker.

In another aspect a method is provided for analysing a biological sample comprising at least a target analyte. The method comprises introducing into the biological sample at least a first marker, or components thereof. The marker comprising an affinity reagent with a backbone specific to the target analyte and a first label with at least a first labelling moiety. The method further comprises generating a first readout of the biological sample with the at least one marker and applying the degradation agent to the biological sample in order to degrade the label oligonucleotide of the marker. The method further comprises introducing at least one second label with at least a second labelling moiety into the biological sample in order to generate a second marker, the second label comprising a second label oligonucleotide configured to hybridise to the barcode oligonucleotide of the affinity reagent, and generating a second readout of the biological sample with the second marker. The method enables iteratively labelling target analytes with several labels. This may be of particular benefit in procedures that aim at detecting a large number of different target analytes in a biological sample. In this case repeatedly imaging the marked biological sample with changing labels may aid correctly identifying the respective target analytes. Preferably, the marker comprises a barcode oligonucleotide resistant to the degradation agent and a label oligonucleotide sensitive to the degradation reagent.

Preferably, the target analyte is identified based on the first label and the second label associated with the target analyte in the first readout and second readout. This enables increasing the probability to correctly identifying or locating the target analyte in the biological sample.

Preferably, in the step of introducing the at least one first marker, the affinity reagent of the at least one first marker is introduced prior to the respective label of the first marker, and wherein the affinity reagent is immobilised in the biological sample prior to introducing the label of the first marker with the first labelling moiety into the biological sample. This enables a particularly robust method since the marker, in particular the affinity reagent, stays in place in close proximity to the respective target analyte even when generating a large number of readouts.

Preferably, the first labelling moiety and the second labelling moiety have different detectable properties. This enables increasing the probability to correctly identifying or locating the target analyte in the biological sample based on the sequence of properties of the labelling moieties detected in the readouts. The detectable properties may be a fluorescent emission, or excitation wavelength, or a emission lifetime, in case of the detectable moiety is a fluorophore, for example.

Preferably, after the step of generating the second readout, the steps of applying the degradation agent to degrade the label oligonucleotide, adding at least one further label with a further labelling moiety, and generating a further readout of the biological sample, are iteratively repeated. This enables increasing the probability to correctly identifying or locating the target analyte in the biological sample based on a large number of readouts. Preferably, for each iteration different labels are used.

It is particularly preferred that the readout generated is an optical readout, for example, by means of a microscope.

### Short Description of the Figures

Hereinafter, specific embodiments are described referring to the drawings, wherein:
- Figure 1: schematically shows a collection of examples of markers bound to target analytes,
- Figure 2: schematically shows how detectable labels may comprise a variety of distinct labelling moieties,
- Figure 3: schematically shows markers with a plurality of labelling moieties,
- Figure 4: schematically shows the use of the present invention for cyclic labelling using barcoded affinity reagents,
- Figure 5: shows a workflow wherein barcoded affinity reagents are introduced into the biological sample and allowed to bind to their targets in a first step,
- Figure 6: shows an optional extension of the workflow depicted in Figure 5, with a second degradation agent,
- Figure 7A: shows a marker with a label with a sensitive barcode oligonucleotide,
- Figure 7B: schematically illustrates how the method may be used to manufacture labels,
- Figure 7C: schematically illustrates how the method may be used to manufacture labels,
- Figure 8: schematically illustrates how the method may be used to mark the same analyte with distinct labels in multiple rounds of a cyclic process (code swapping),
- Figure 9: schematically illustrates *in situ* assembly of a marker,
- Figure 10: schematically illustrates the use in affinity purification and affinity capture-based assays,
- Figure 11A: shows a marker with a barcode oligonucleotide comprising 3 barcodes (predetermined sequences) and a single-colour fluorescent label,
- Figure 11B: shows a marker with a barcode oligonucleotide comprising 3 barcodes (predetermined sequences) and a multi-colour fluorescent label (combinatorial label or combinatorial fluorescent code, CFC),
- Figure 11C: shows a marker with a barcode oligonucleotide comprising 1 barcode (predetermined sequences) and a polyyne label, and
- Figure 11D: shows a marker with a barcode oligonucleotide comprising 1 barcode (predetermined sequences) and a polyyne-fluorescent combinatorial label.

### Detailed Description

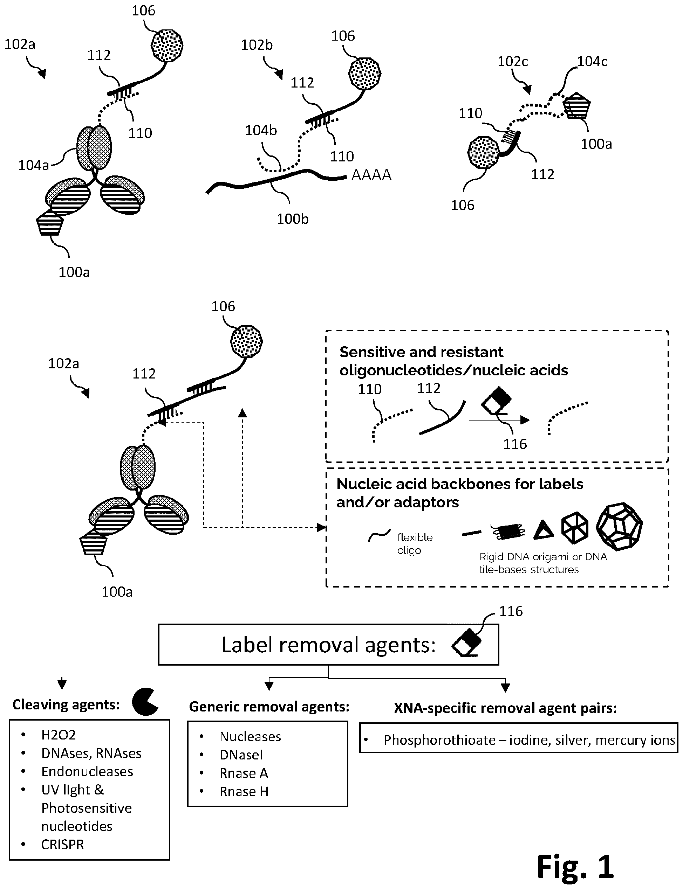

Figure 1 shows a collection of examples of markers 102a, 102b bound to their respective target analytes 100a, 100b. The markers 102a, 102b, 102c may comprise an affinity reagent with a backbone, for example an antibody 104a, an oligonucleotide probe 104b, which may comprise a naturally occurring nucleic acid or a non-naturally occurring nucleic acid (XNA), or an aptamer, aptabody, or otherwise modified aptamer 104c. Furthermore, the affinity reagent backbone may be based on another non-naturally occurring polymer and non-naturally occurring residues and/or modifications.

The markers 102a, 102b, 102c comprise the respective affinity reagent backbones 104a, 104b, 104c. The markers 102a, 102b, 102c further comprise labels with a labelling moiety, for example an optically detectable fluorophore 106. The labels comprise an oligonucleotide with at least a label oligonucleotide 112. The affinity reagent similarly comprises an oligonucleotide with at least a barcode oligonucleotide 110, this combination may be termed a barcoded affinity reagent. The label oligonucleotide 112 and the barcode oligonucleotide 110 are configured to specifically hybridise to each other. Thus, the label may be attached specifically to the respective affinity reagent with antibodies 104a, 104b, 104c to generate the markers 102a, 102b, 102c based on the specificity of the respective label oligonucleotides 112 and the barcode oligonucleotides 110. The label oligonucleotide 112 may be attached to the fluorophore 106 of the label covalently. Similarly, the barcode oligonucleotide 110 may be attached to the backbone of the affinity reagent covalently.

In the sense of this document a marker 102a, 102b, 102c refers to the physical association of affinity reagent and detectable label or to the virtual assignment or mapping of affinity reagent to detectable label. A marker 102a, 102b, 102c may be physically constituted before, during, or after introduction into a biological sample. Likewise, a detectable label may be physically constituted before, during, or after introduction to a biological sample. A biological sample may be any of the following: a cell, a cell culture (monolayer, suspension, 3D cell culture, spheroid, organoid, tissuoid), a tissue section (e.g. FFPE, cryosections), a solid biopsy, a liquid biopsy, a sample of a bodily liquid (e.g. blood, serum, plasma, sperma, sputum, liquor, lympha, intersitial fluid). Such a biological sample may comprise at least one target analyte of interest. Such a target analyte 100 may be a protein 100a or a mRNA 100b for example.

For the method disclosed in this document barcoded affinity reagents are used, i.e. antibodies, nanobodies, ISH probes, aptamers, comprising the barcode oligonucleotide 110, which comprises at least one unique sequence that is assigned to said affinity reagent uniquely. Said barcode oligonucleotide 110, may comprise multiple barcode sequences, which may be addressed by multiple detectable labels, in particular label oligonucleotide 112, either simultaneously or in a temporally sequential manner. An affinity reagent in the sense of this document may also be any molecule that exhibits high affinity to a target analyte and binds to said analyte in a substantially specific manner. In the sense of this document, a drug, drug-like molecule, a small molecule, or a toxin may also be used as an affinity reagent and conjugated to a barcode oligonucleotide 110.

Said detectable label, therefore comprises the label oligonucleotide 112, which comprises at least one barcode sequence which is configured to be substantially complementary and thus able to specifically bind to said barcode sequence of the barcode oligonucleotide 110 of said assigned affinity reagent, to allow the flexible connection of affinity reagent and detectable label, i.e. marker formation.

Said label oligonucleotide 112 in the sense of this document may be a short dye-conjugated oligonucleotide, a long flexible oligonucleotide comprising barcode sequences for the attachment of a third label-conjugated oligonucleotide for example, or may even comprise a 2D-/3D-DNA-based structure like a DNA origami or DNA brick based structure.

Said barcode oligonucleotide 110 is preferably configured such that it is in its entirety or at least with respect to the interaction-mediating portions (i.e. barcodes mediating the connection between affinity reagent and detectable label) resistant to a degradation agent 116.

Said label oligonucleotide 112 is preferably configured such that it is in its entirety or at least with respect to the interaction-mediating portions (i.e. barcodes mediating the connection between affinity reagent and detectable label) sensitive to the degradation agent 116. Sensitive means the degradation agent 116 can bring about a change in the label oligonucleotide 112 sufficient to allow label removal. This may be a nicking of the barcode at a central position, that triggers dissociation of the remaining halves from the barcode oligonucleotide 110. This may be a partial digest of the barcode sequence, a complete digest of the barcode sequence or even include a partial or complete degradation of other components of the detectable label.

For example, the barcode oligonucleotide 110 may comprise PT-modified DNA, or LNA, or PNA, or morpholino, or another XNA-resistant to DNase I, this is schematically depicted in the Figure 1 as a dotted line. Such a barcode oligonucleotide 110 can be easily conjugated to an antibody for example by using commercially available conjugation kits like the Oligonucleotide Conjugation Kit (ab218260) from Abcam (Cambridge, United Kingdom).

Thus, the degeneration sensitive label oligonucleotide 112 (depicted as a full line) may be a naturally occurring nucleic acid such as DNA, which is sensitive to degeneration by the DNase I degeneration agent 116. The degeneration resistant barcode oligonucleotide 110 (depicted as a dashed line) may be nucleic acid analogue, such as a non-naturally occurring nucleic acid (XNA) such as L-DNA, which is resistant to degeneration by the DNase I degeneration agent 116. In case of the nucleic acid based affinity reagent backbone 104b, the backbone 104b is preferably equally of a nucleic acid analogue such as non-naturally occurring nucleic acid such as L-DNA resistant to degeneration.

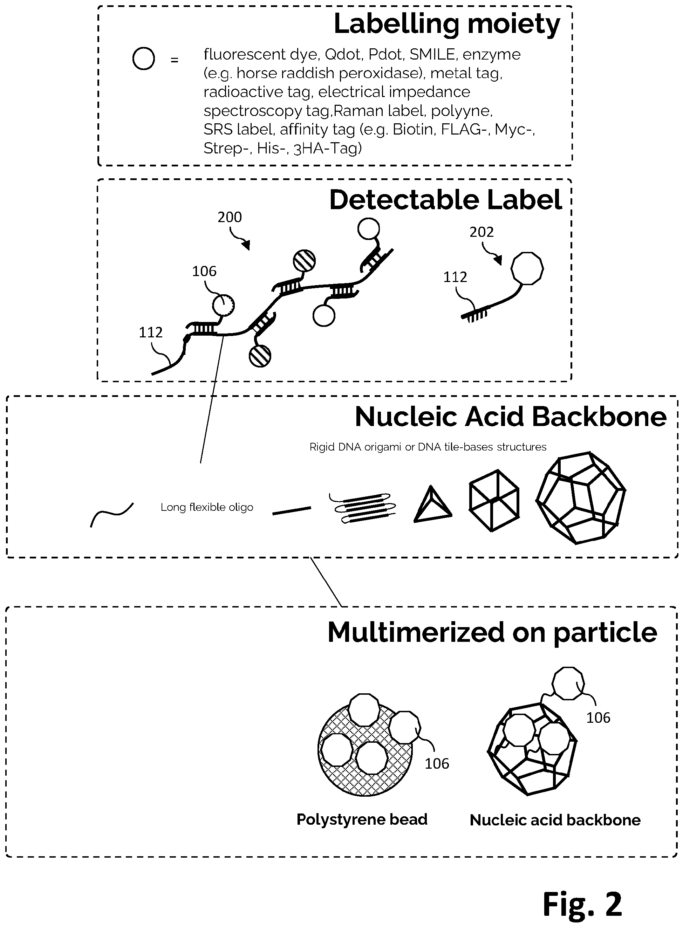

Figure 2 schematically shows how detectable labels 200, 202 in the sense of this document may comprise a variety of distinct labelling moieties for various applications including optical or non-optical detection, as well as imaging or non-imaging based readout. A labelling moiety may be conveniently connected to either a second or third oligonucleotide. In Figure 2 a third (or fourth,...) oligonucleotide is conjugated covalently to the labelling moiety. Click-chemistry allows efficient generation of such conjugates but other chemistries are equally suitable (e.g. NHS coupling). Also affinity-interaction based coupling of labelling moieties via high affinity interactions (biotin-Streptavidin; curcubit uryls [CB] like CB[7] or CB[n] homologues and high-affinity guests (for example PubChem ID 101402794), an expanded cubane described in Lambert et al. 2019) is a suitable strategy to connect labelling moieties with backbones of the detectable label. Such backbones may then comprise further DNA-/XNA-based structure or non-DNA particles like polystyrene beads 204.

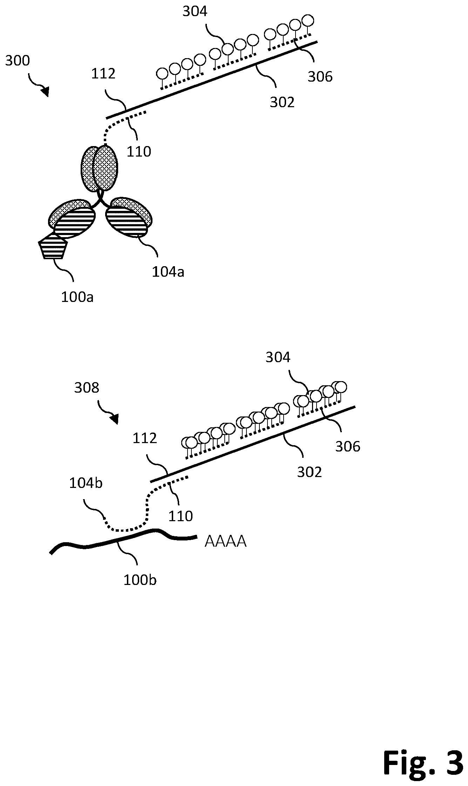

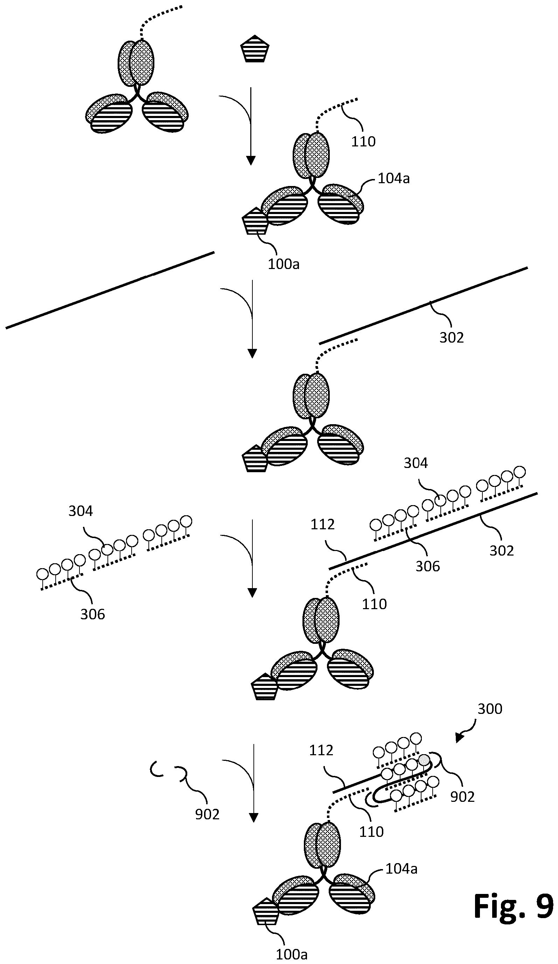

Figure 3 shows a particularly preferred embodiment of a marker 300 with a label comprising a long oligonucleotide 302 used as an adapter onto which a plurality of labelling moieties 304 bearing oligonucleotides 306 are hybridised. The long oligonucleotide 302 comprises the label oligonucleotide 112 for specifically hybridising to the barcode oligonucleotide 110 connected to the antibody 104a. Similarly, a nucleic acid based marker 308 may comprise the long oligonucleotide 302. Figure 9 further shows, how this embodiment may also be used to assemble the detectable label *in situ.* The assembly in situ provides numerous advantages in terms of penetration into the sample for example, or with respect to reducing production cost.

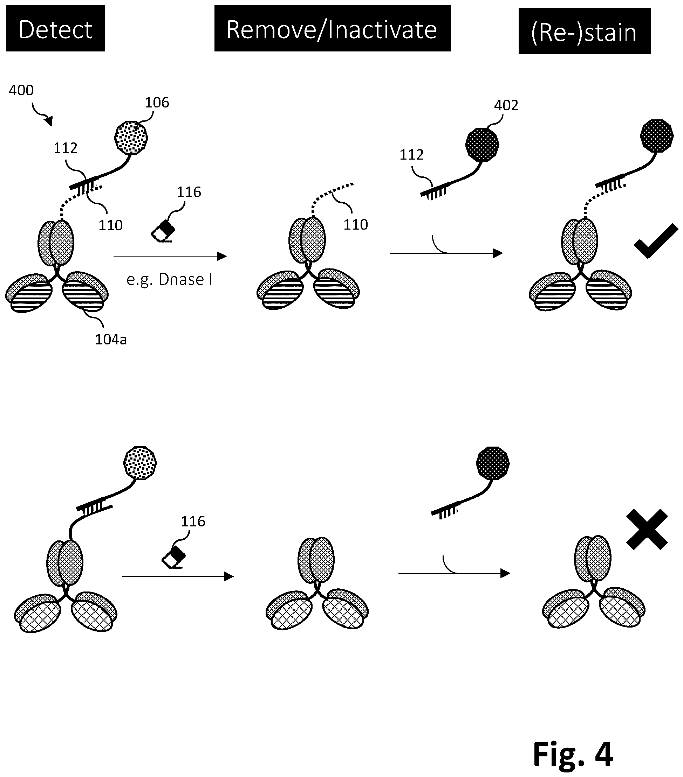

Figure 4 schematically shows the use of the present invention for a cyclic labelling using barcoded affinity reagents. The use of the present invention in conjunction with DNase I as a degradation agent 116 is particularly preferred, as DNase I is very robust, allows for fast (i.e. catalytic) removal, and is easy and inexpensive to produce. The top view of Figure 4 shows a marker 400 with a label with a labelling moiety 106 which is removed from the barcode oligonucleotide 110 by degrading the label oligonucleotide 112 with the degradation agent 116. In a next step a further label comprising a labelling moiety 402 different to the labelling moiety 106 may be attached to the barcode oligonucleotide 110.

This is contrast to the alternative shown in the bottom view of Figure 4, where the oligonucleotides connecting the label and the affinity reagent are both sensitive to the degradation agent 116. In this case, after applying the degradation agent 116 both oligonucleotides are degraded and no further labels may be attached to the affinity reagent.

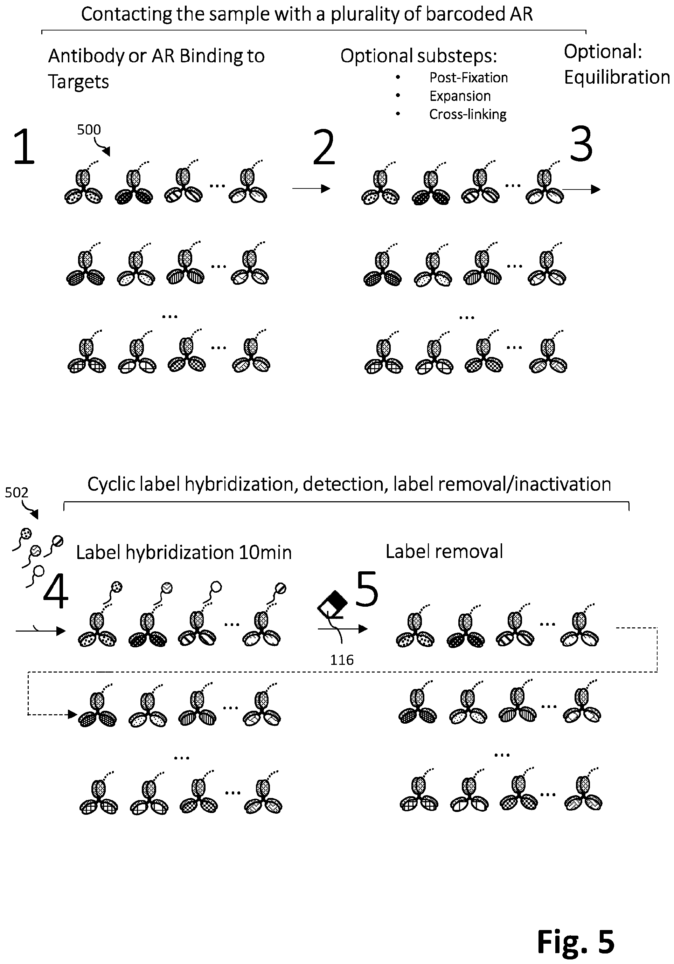

Figure 5 shows a workflow wherein a plurality of barcoded affinity reagents 500 are introduced into a biological sample and allowed to bind to their respective target analytes in a first step (indicated by the "1"). For the sake of simplicity this is shown in

Figure 5 exemplarily for antibodies but meant to be understood as generally applicable to any affinity reagent. For example, the affinity reagents may comprise in situ hybridization probes, aptamers, aptabodies, or nanobodies. Furthermore, the plurality of affinity reagents 500 may comprise any of the aforementioned classes, i.e. may be of a mixed composition with respect to type or class of affinity reagent backbone.

Likewise, for the sake of simplicity the targets are not shown in Figure 5. Following optional steps 2 and 3 (indicated by "2" and "3"), wherein the sample may undergo further post-fixation, expansion, and/or cross-linking, a first plurality of detectable labels 502 with sensitive label oligonucleotides is introduced into the biological sample and allowed to bind specifically to the resistant barcode oligonucleotides of the respective affinity reagent (indicated by "4"). Once one of the labels 502 hybridises to one of the affinity reagents 500 a respective marker is generated. Optionally any unbound labels 502 are then washed out. The bound labels are then readout by the readout device, for example by fluorescent microscopy in case the labelling moieties of the labels 502 are fluorophores. Thus, the marker enables the identification and localisation of the target analyte the respective marker is bound to by detecting the associated label of the marker, in particular the labelling moiety of the label. In a subsequent step 5 (indicated by "5") the degradation agent 116 is applied, the label oligonucleotides of the labels are degraded and removed from the affinity reagents 500, and the remaining components of the labels are removed from the biological sample by washing out. Steps 4 to 5 (including respective readouts) are then repeated until barcoded affinity reagents of the introduced plurality of affinity reagents 500 (Step 1) have been labelled and readout at least once. Thus, when repeating the steps 4 and 5, not all the affinity reagents of the plurality 500 need to be labelled by one of the introduced labels 502 in a particular step 4 and 5. Rather, the number of distinguishable labels 502 that may be generated determines the number of markers that may be generated from the affinity reagents 500 and the labels 502 in any one step 4.

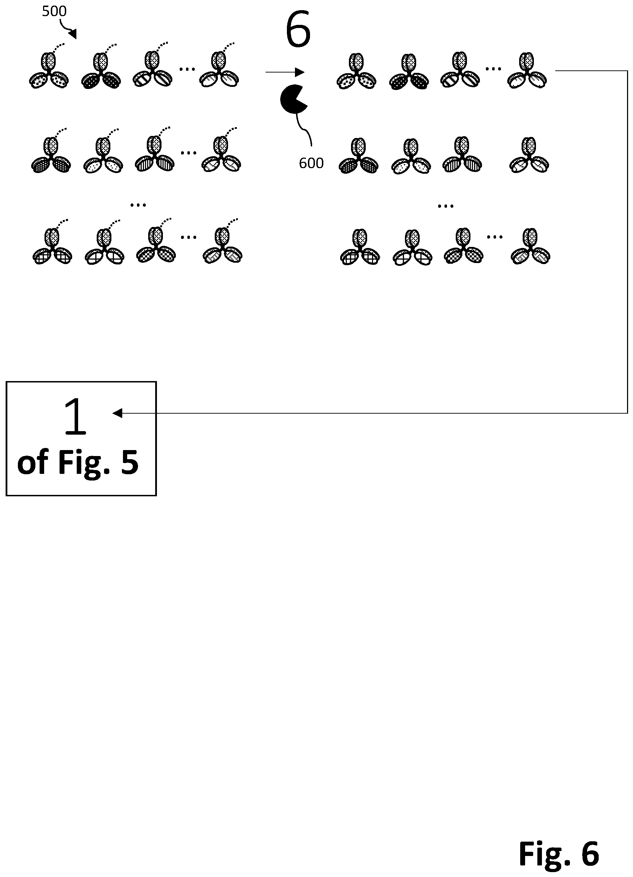

Figure 6 shows an optional extension of the workflow depicted in Figure 5. In particular, the steps shown in Figure 6 may follow step 5 described for Figure 5. Specifically, a second degradation agent 600 is used in Figure 6, to remove all barcode oligonucleotides. Thus, the barcode oligonucleotides of the affinity reagents may be sensitive to the second degradation agent 600, but not the first degradation reagent 116. Such a reset of the biological sample may be desired, if only a limited number of physical barcodes is available. For example, if only 50 barcodes physically exist and the user wants to analyse 200 markers, then this allows the method to be performed in 4 cycles, wherein each cycle contains 50 markers and is performed according to Figure 5 using a given number of subcycles. After each subcycle, the barcode oligonucleotides of the markers 500 used in the subcycle are removed by addition of the second degradation agent 600 and a further subcycle according to Figure 5 is initiated with the next plurality of 50 markers, for example.

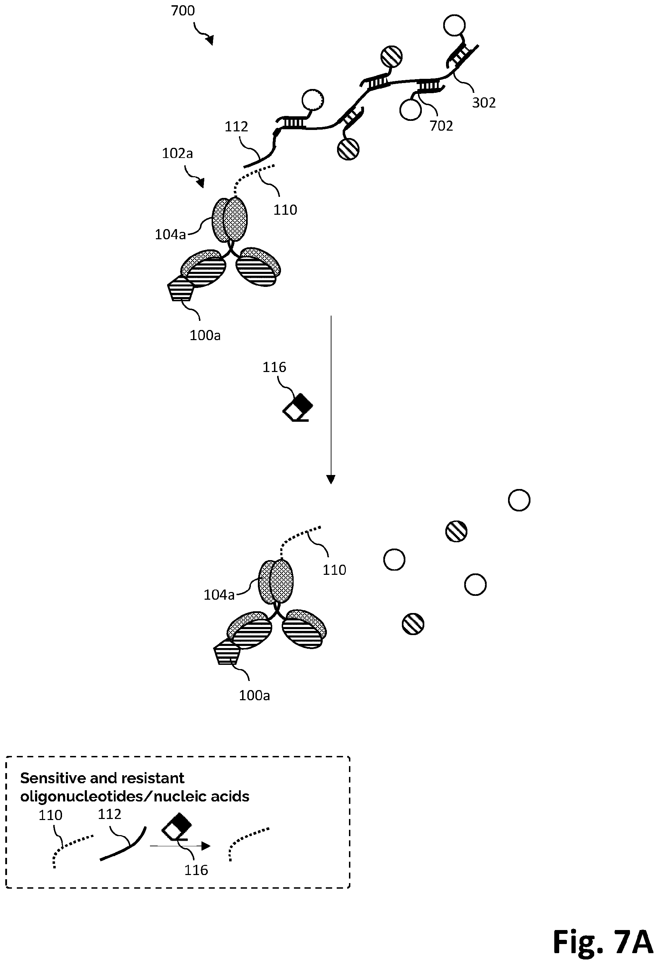

Figure 7A to 7C schematically depict different label removal mechanisms in combination with various types of labels, e.g. single-color, multi-color (combinatorial), Raman (e.g. polyynes), and hybrid labels comprising more than one modality e.g. fluorescence and Raman labelling moieties.

Figure 7A shows a label 700 with the sensitive label oligonucleotide 112 and a sensitive long oligonucleotide 302, and sensitive oligo-dye conjugates 702. In this case the degradation agent 116 leads to the complete disintegration of the detectable label 700.

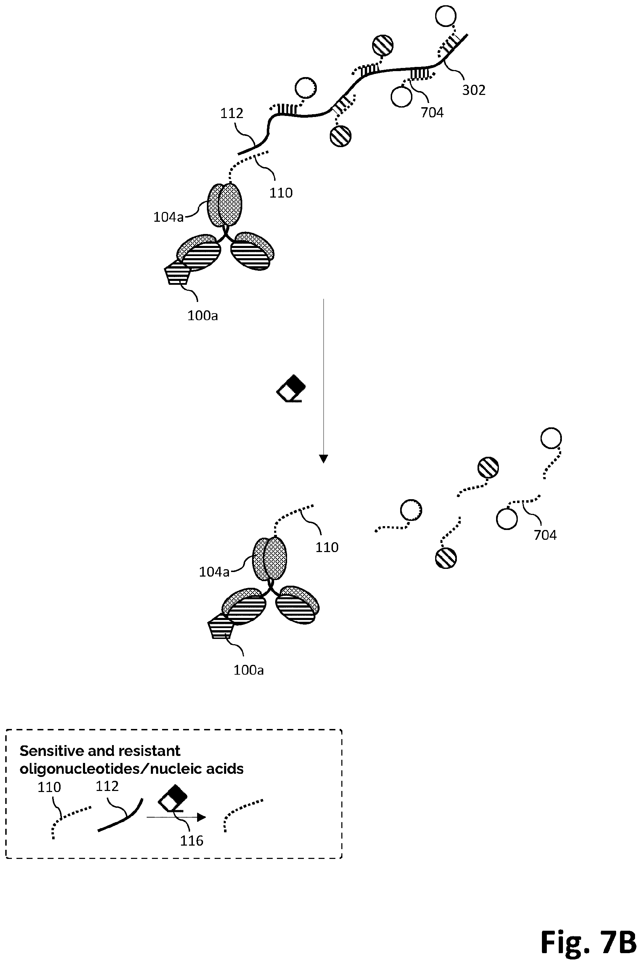

Figure 7B differs from 7A in that here resistant dye-oligo conjugates 704 connect dyes to the sensitive long oligonucleotide 302 and the sensitive label oligonucleotide 112. The disintegration by the degradation agent 116 is thus incomplete. The resistant dye-oligo conjugates 704 are, however, still small and can be efficiently removed from the sample. In this example PNA-dye conjugates may mediate strong connection to for example the sensitive long oligonucleotide 302 comprising DNA, which connects to the barcode oligonucleotide 110 comprising PNA or PT-modified DNA via the label oligonucleotide. This is particularly, advantageous because DNA/PNA interaction is more specific and thus has better signal-to-background because of lower cross-hybridization. Further, only a portion of the detectable label, i.e. the label oligonucleotide 112 may be sensitive, i.e. only the label oligonucleotide 112 comprises DNA and the rest comprises for example PNA.

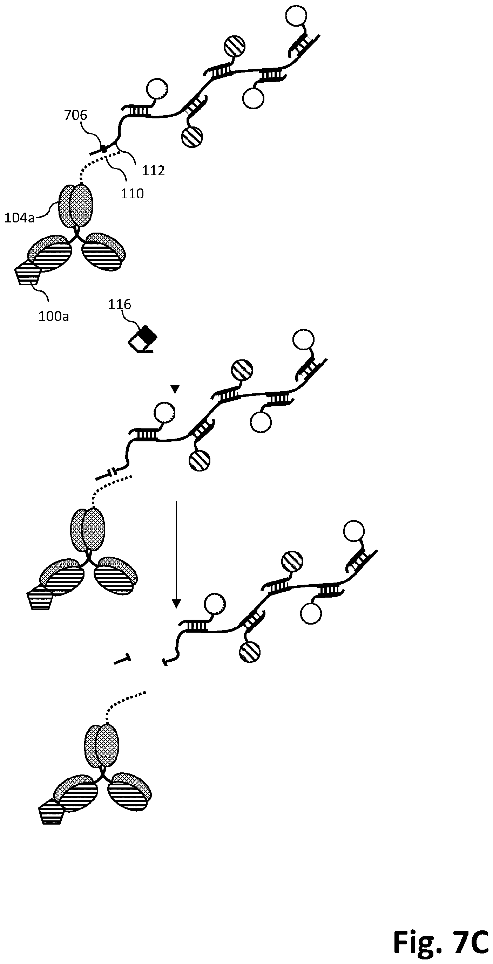

Alternatively, as depicted in Figure 7C both barcode and label oligonucleotide 110, 112 may comprise the same backbone DNA, RNA, or XNA, but the label oligonucleotide 112 comprises a point modification/mutation 706 that is recognized by structure-specific enzyme as degradation agent that introduces a nick at this position. Introduction of the nick results in degradation of the label oligonucleotide 112. Alternatively or in addition, CRISPR Cas9 or a derivative, for example Cas9, or Cas9 H840A nickase may be used to cut the sensitive label oligonucleotide 112, in this case a suitable guide RNA or guide LNA would be used to target CRISPR/Cas9 to the respective sequence.

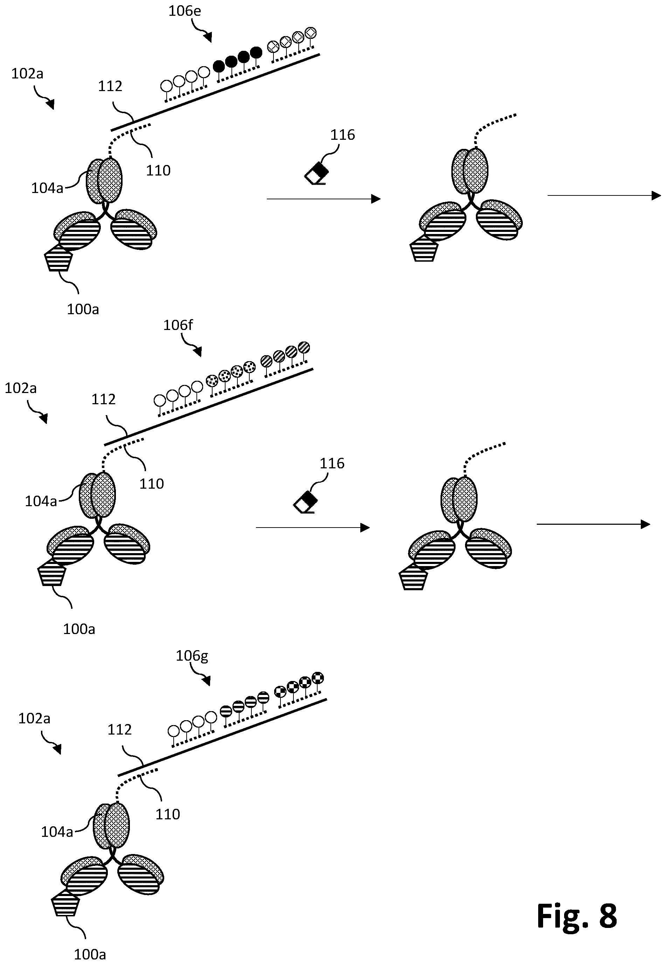

Figure 8 schematically shows, how the present invention may be used to mark the target analyte 100a in an n-th round with a n-th label 106e (n=1 in the example of the top row in Fig. 8), perform a readout, remove the label 106e and relabel again with a second label 106f binding to the same affinity reagent with antibody 104a, either on the same barcode oligonucleotide 110 or on another barcode oligonucleotide 110. In this way, the target analyte 100a is marked with the respective labels 106e, 106f, 106g in a first, second, third round. This method of labelling the same target analyte 100a with multiple markers over multiple rounds in a temporally sequential fashion using barcoding may be referred to as barcode cycling. This is a particularly preferred, mode of using the invention to perform "code swapping" in the sense of the publication application WO 2022/242887 A1, which discloses a method for high-plex analysis.

Figure 9 schematically depicts the formation of the marker 300 and the formation of associated the detectable label in situ, i.e. in the sample. Assembly may be performed by simultaneous introduction of all components of the marker 300, or by sequential introduction as shown in Figure 9. The label may further be compacted using the method disclosed in the European patent application with the application number EP 23174843, the complete content thereof being incorporated by reference herein. To that end staple strands 902 are added to the long oligonucleotide 302, the staple strands 902 hybridising to respective binding sites of the long oligonucleotide 302 in order to fold the long oligonucleotide 302 into a compact shape.

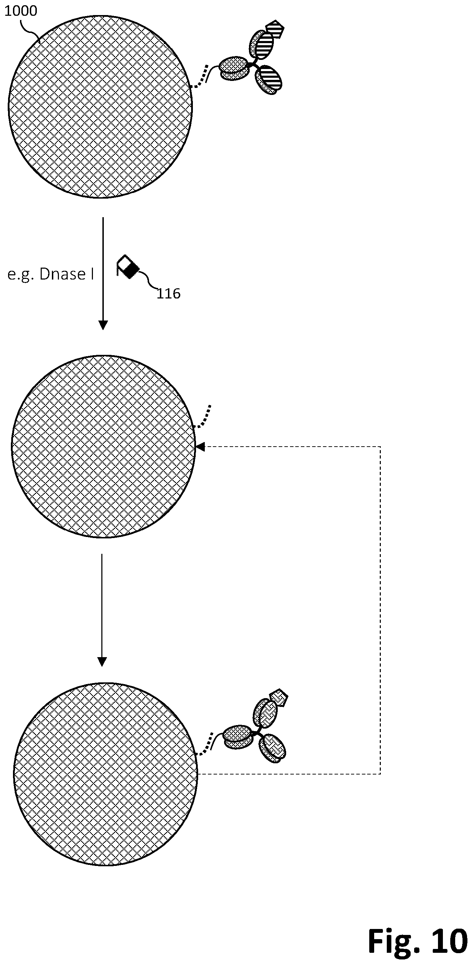

Figure 10 shows a schematic of an affinity purification using a column matrix like polystyrene beads 1000, which are connected to a capture affinity reagent, which is barcoded. In this case the method may be used to remove the bound affinity reagent:target complex by label oligonucleotide or barcode oligonucleotide removal. This may also be used to regenerate the column matrix.

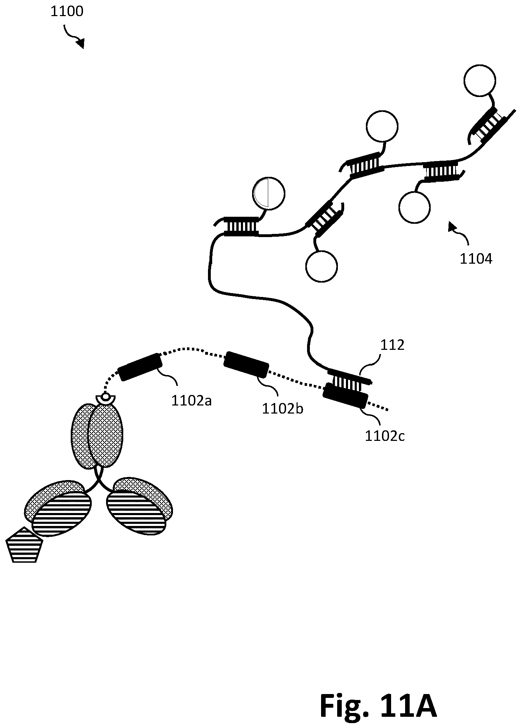

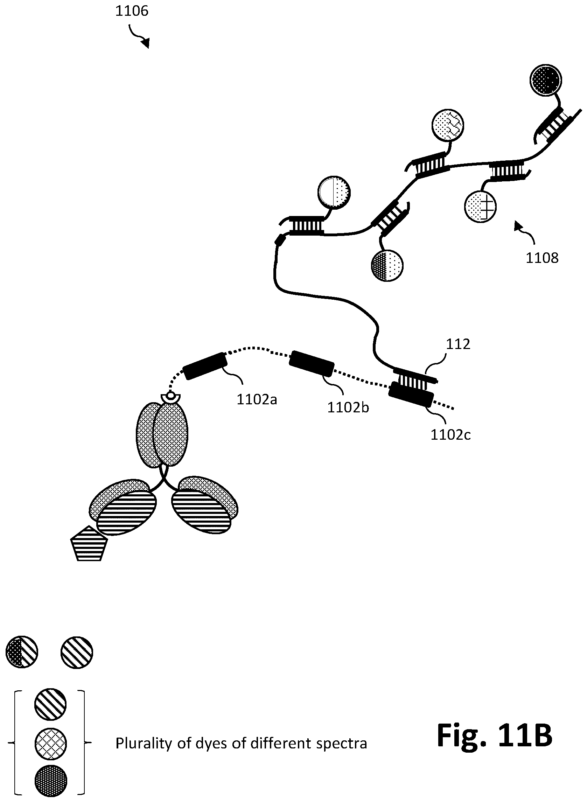

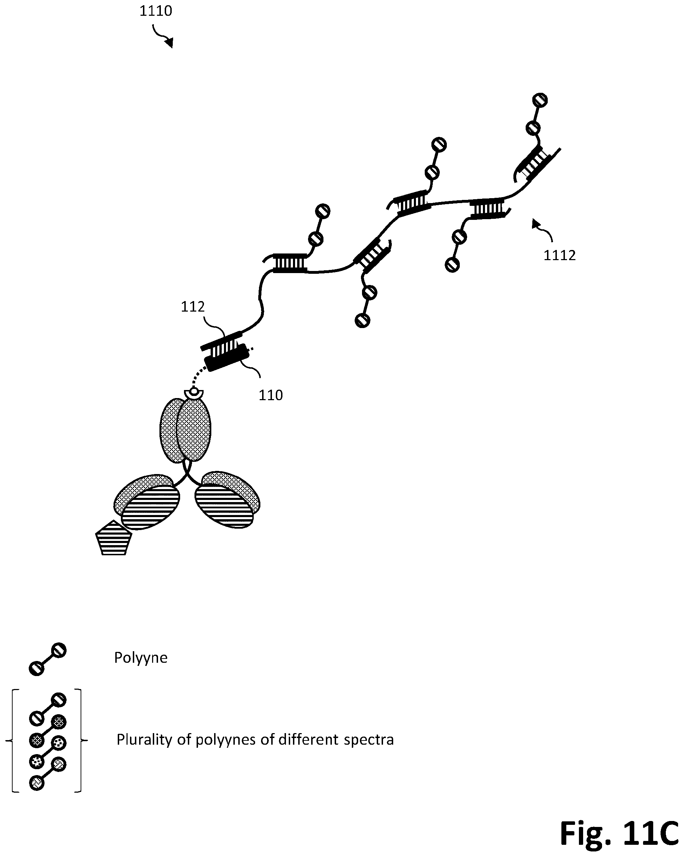

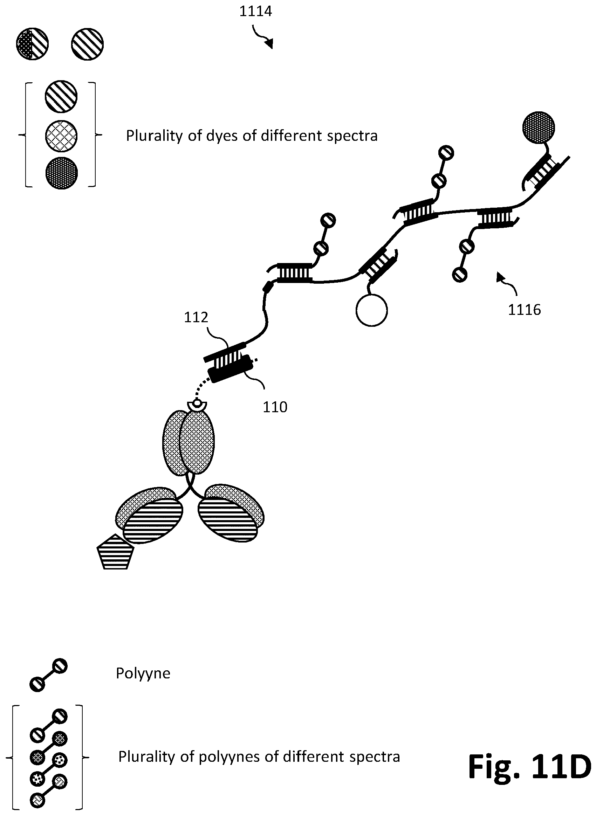

Figure 11A to 11D show schematically various exemplary embodiments of markers. For example, Figure 11A shows a marker 1100 comprising three barcode oligonucleotide sequences 1102a, 1102b, 1102c for attaching labels and a single-colour fluorescent label 1104. Figure 11B shows a marker 1106 comprising the three barcode oligonucleotide sequences 1102a, 1102b, 1102c and a multi-colour fluorescent label 1108 (combinatorial label or combinatorial fluorescent code, CFC). Figure 11C shows a marker 1110 with the barcode oligonucleotide 110 and a label 1112 comprising polyyne labelling moieties. Figure 11D shows a marker 1114 with the barcode oligonucleotide 110 comprising a polyyne-fluorescent combinatorial label 1116.

The use of barcoded affinity reagent backbones, for example antibodies, nanobodies, aptamers, in situ probes targeting DNA or mRNA, which are connected to an oligonucleotide comprising at least one sequence also referred to as a barcode that is unique to said affinity reagent. This oligonucleotide-labeled affinity reagent may be referred to as a barcoded affinity reagent. Barcoding of affinity reagents has numerous advantages over direct-conjugation of for example fluorescent dyes as it allows the flexible use of a barcoded antibody for example with a variety of detectable labels in different applications or in different rounds of a cyclic process of the same application. A detectable label may refer to optically detectable labels used for microscopy, cytometry, or spatial biology applications as well as to non-optically detectable labels, which may be used for affinity purification, cyclical western blotting, ELISA- and ELISPOT-assays, high-plex proteomics (e.g. plasma proteomics). A detectable label thus comprises at least one labeling moiety, which may be a fluorescent dye, an affinity tag, metal tag, or enzyme for example. A detectable label may further comprise at least a label oligonucleotide comprising a sequence configured to bind to a complementary barcode oligonucleotide of an assigned barcoded affinity reagent. The label oligonucleotide, may thus be a simple short oligonucleotide attached to a label, for example a dye-conjugated oligonucleotide, or a more elaborated uni- or multipartite structure. The latter may be for example a long flexible oligonucleotide backbone, a dendrimeric nucleic-acid based backbone, or a rigid DNA-based backbone with a 2-dimensional/3-dimensional structure (e.g. DNA origami, DNA brick-based), or a backbone composes of a nucleic acid component and a non-nucleic acid component for example an oligonucleotide attached to a dye-conjugated polystyrene bead.

The barcode and label oligonucleotide may comprise one or multiple predetermined or barcoded sequences.

### Cyclic staining

The barcoding of affinity reagents and detectable labels is an effective strategy for cyclical labeling, which may be used for example in multiplexed biomarker, high-plex RNA imaging or high-plex spatial or non-spatial proteomics applications. Likewise, it is a prerequisite for of high-plex spatial interactomics or the interrogation of protein modifications at high plexity.

### Marker cycling

In the sense of cyclic staining, barcoded affinity reagents may be used in different modes: For example marker cycling, wherein in a cyclic staining, imaging, inactivation process markers are introduced to the biological sample in every round and allowed to bind to their targets. In this case markers are pre-assembled and each round includes a marker introduction step, which is time-consuming.

### Label cycling

Further, said barcoded affinity reagents may be used for label cycling, this is a particularly preferred mode of operation in the sense of the present invention. In this mode, all barcoded affinity reagents from a plurality of barcoded affinity reagents are introduced to the sample in a first step, and then in subsequent steps a subset of assigned barcoded detectable labels is introduced to the sample, allowed to bind to the assigned barcoded affinity reagents, readout and inactivated or removed. The process is then repeated in a cyclic fashion until all barcoded affinity reagents from said plurality of barcoded affinity reagents are readout for example. Label cycling in contrast to marker cycling, does not necessitate repeated introduction of affinity reagents and therefore requires substantially less time, as the introduction and binding of affinity reagents to their targets may typically take between 1h-24h. This strategy offers the advantage that is all antibodies for example may be bound to their analytes in a single step followed by a post-fixation and then these antibodies may be labeled in a cyclical in situ hybridization using barcoded labels over multiple rounds. As the binding of antibodies to their targets requires about 1-3h when tissue sections are stained and label hybridization may require about 10min, this allows for increased throughput. In order to remove bound labels several strategies may be employed like cleaving with chemical agents like TCEP, which requires disulfide bridge modified oligonucleotide barcodes labels, melting, or strand displacement.

### Barcode cycling

Another strategy that can be used with barcoded affinity reagents is a variety of label cycling, to which we refer to as barcode cycling in this document. In this case the same barcode is addressed multiple times with different detectable labels, which may be enabled by choosing a configuration, wherein barcodes of affinity reagents and detectable labels are configured such that they only transiently associated like it is the case for example with DNA-PAINT, or in a cyclical process where binding is controlled by buffer conditions (e.g. DNA Exchange imaging), temperature (e.g. melting), absence/presence of displacement strands, or strand-invasion of guest-host complexes.

### Label removal mechanisms

A degradation agent, can be used to selectively bring about the removal of a oligonucleotide configured to be sensitive to the degradation agent, and thereby allow dissociation of the detectable label carrying the sensitive label oligonucleotide from the marker. This entails that the degradation agent is not necessarily required to degrade the sensitive oligonucleotide portion completely, but in a manner sufficient to allow dissociation of the detectable label from the bound barcoded affinity reagent. The degradation agent and sensitive oligonucleotide are configured such that the degradation agent clears the resistant barcode effectively from the bound sensitive complementary barcode, such that said resistant barcode of the barcoded affinity reagent is free to rehybridize with a different detectable label in a subsequent round.

When nucleic acid-based labels are used that comprises for example a nucleic acid-based backbone, then degradation agents may in addition allowing removal of the detectable label bring about degradation of the label, this may facilitate rapid inactivation and washing out the broken-down components of the detectable label from the biological sample.

The degradation agent may thus
- Introduce a nick into the sensitive oligonucleotide causing the dissociation of the hybridized label and barcode oligonucleotide.
- Substantially degrade the sensitive oligonucleotide or substantially degrade the sensitive portion.
- Partially degrade the sensitive oligonucleotide or partially degrade the sensitive portion in a manner suited to release the resistant barcode and allow label removal.
- Partially or substantially degrade parts of the detectable label including for example the labeling moieties and/or dye conjugated oligonucleotides.

### Suitable degradation agents

The core idea is to use a degradation agent and at least two distinct nucleic acids, i.e. the label and barcode oligonucleotides, selected from the group of naturally occurring nucleic acids or nucleic acid analogues such as artificial/xeno nucleic acids (XNA), wherein the pairing comprises at least one sensitive and one resistant oligonucleotide. It is therefore important to note that sensitive and resistant is an attribute that is in any case related to the selected degradation agent, which may be selected from one of the following:
- Endonucleases
- Restriction endonucleases/restriction enzymes
- AP endonucleases
- DNA-repair enzymes and structure-specific endonucleases
- Endonuclease III (Nth), Endonuclease IV
- APE 1
- RNase HII (e.g. nicks ribonucleotides incorporated into a DNA duplex)
- RNA endonucleases
- RNase A, RNase T1, and RNase H
- Drosha and Dicer
- Exonucleases
- Exol, Exolll, ExoV, Lambda Exonuclease, T5 Exo, ExoV, ExoVll, ExoT, T7 Exo
- RNA Exonucleases RNase D, RNase T
- Nuclease P1, Mung Bean Nuclease, RecJfr, DNase I, Micrococcal Nuclease
- CRISPR and related enzymes
- Artificial enzymes
- Engineered artificial peptidase or protease that degrades PNA backbones
- Organic or inorganic chemistries to which at least one sensitive and at least one resistant nucleic acid can be found
- Iodine, silver ions (e.g. cleave phosphorothioate modified DNA)

In this sense, it is clear that many combinations of enzymatic or non-enzymatic degradation agents with a sensitive nucleic acid and a resistant nucleic acid can be found, that may be used to implement embodiments of the present invention. Furthermore, it is clear that one nucleic acid, for example, phosphorothioate modified DNA is resistant to a first degradation agent for example DNase I, but sensitive to a second degradation agent, for example silver or iodine ions. A further example of an inorganic chemistry is cleaving of phosphorothioate (PT) modified oligonucleotides with iodine or silver for example. Multiple PT cleavage sites may be incorporated into the barcode or backbone of the label and iodine and/or silver may be used as a removal agent.

This enables configurations in which the sensitivity and resistance of the barcode and label oligonucleotide, can be reversed during an experiment or process by changing from a first to as second degradation agent. In the sense of a cyclic imaging process, this may for example be used to perform label cycling with label removal as described above, wherein the label oligonucleotide of the detectable label is sensitive to a first degradation agent, in a first phase of the process and then all barcode oligonucleotides of the barcoded affinity reagents are removed in a second phase by a second degradation agent. This may be of use, in situations where the total number of available barcode sequences is limited for example when there are only 50 physically available barcodes, and 200 markers shall be analysed, then this may be performed in 4 phases of label cycling, wherein after each phase the barcodes of the affinity reagents are removed.

Likewise, both the label and the barcode oligonucleotides, may be sensitive against a second degradation agent.

### Suitable nucleic acids and xeno nucleic acids (XNA)

Suitable nucleic acids or xeno nucleic acids (XNA) from which pairs of sensitive and resistant oligonucleotides or nucleic acids may be selected:
- locked nucleic acid (LNA),
- morpholino,
- phorphorothioate modified nucleic acid,
- peptide nucleic acid (PNA),
- bridged nucleic acid (BNA),
- 2'O-methyl-stubstituted RNA,
- glycol nucleic acid (GNA),
- hexitol nucleic acid (HNA),
- altritol nucleic acid (ANA),
- threose nucleic acid (TNA)
- Cyclohexene nucleic acids (CeNA),
- fluoroarabinonucleic acid (FANA)
- D-DNA
- L-DNA
- RNA
- Duplexes of any DNA, RNA, XNA combination, wherein one part of the duplex contains at least one modification or modified base, which is recognized by structure-specific enzyme

Sensitive barcode oligonucleotides and/or sensitive label oligonucleotides may comprise: DNA, RNA, any XNA that is substantially sensitive to the removal agent, i.e. is cleaved at multiple sites, which leads a corresponding oligonucleotide to degrade in the presence of the removal agent or a corresponding label oligonucleotide of the label to substantially degrade and release the label from the affinity reagent.

In a particularly preferred embodiment, the barcoded affinity reagent comprises the barcode oligonucleotide 110, which is resistant to the degradation agent 116, and the detectable label comprises the label oligonucleotide 112, which is sensitive to the degradation agent 116.

In a particularly preferred embodiment, the degradation agent 116 is DNase I and the barcoded affinity reagent comprises the barcode oligonucleotide 110, which is resistant to the degradation agent 116, comprising for example a PNA, LNA, PT-modified DNA, GNA, FANA, or backbone, and the detectable label comprises the label oligonucleotide 112, which is sensitive to the degradation agent 116 comprising for example a DNA backbone.

In a particularly preferred embodiment, of the present invention DNase I is used as a degradation agent 116, and the barcoded affinity reagent comprises the barcode oligonucleotide 110, which is resistant to the degradation agent.

In a particularly preferred embodiment of the present invention, effective removal of labels is performed using DNase I, while leaving the barcodes on the affinity reagents intact. The resistant barcodes in this case may comprise any XNA that is resistant to DNase I for example a PNA or phosphorothioate modified (PT) DNA. The benefit is a fast, robust, and cost-effective way to use label removal with barcoded affinity reagents.

In addition to enabling a fast and cost-efficient removal process that is based on DNase I in conjunction with label cycling via hybridization the present method provides another advantage. When barcoded affinity reagents are used to stain analytes in a biological sample and detectable labels are bound to them via hybridization then cross-hybridization of labels to OFF-barcodes may occur and generate false-positive results. It is therefore desirable to improve the specificity of binding, which is may be done by increasing the temperature under which hybridization is performed. This may, however, lead to background formation in the sample. The usage of hybrid configurations like XNA:DNA or a first XNA:a second XNA that is proposed in this document also may improve specificity of barcode recognition substantially under conditions (e.g. temperature, ionic strength, pH) that are typically being used for staining.

It is known for example that PNA:DNA hybridization is more stringent than DNA:DNA hybridization. PNAs are very stable and resistant to both proteases and nucleases. Similarly, morpholinos or LNA are known to bind to their targets with great specificity. PNA probes have been used successfully for single molecule fluorescent in situ hybridization and shown to provide higher specificity than DNA probes. This is due to the higher affinity of PNA oligonucleotides with respect to DNA oligonucleotides of the same length.

In a particularly preferred embodiment, of the present invention the detectable label therefore comprises PNA-oligonucleotides comprising covalently conjugated labeling moieties such as for example fluorescent dyes, polyynes or other Raman labels. PNA probes for smFISH or dye-conjugated PNA oligonucleotides with 1, 2, 4, or n directly conjugated dye molecules, wherein the direct conjugation may be at the 2' position of the sugar backbone, are commercially available from for example (Biomers.net GmbH, Ulm, Germany). Conjugation of labeling moieties may be conveniently performed using click chemistry. For example, using Click-FISH PNA oligonucleotides from (Biomers.net GmbH, Ulm, Germany), it is possible to conjugate labeling moieties via Azide-Alkyne click chemistry. It is therefore possible, to conjugate a wide variety of labeling moieties quickly and easily to a PNA oligonucleotide. Lablling moieties may be:
- optically detectable labels such as fluorescent dyes, PDots, QDots, SMILEs, optically readable semiconductor particles or Raman labels (e.g. polyynes), or
- sequenceable labels such as oligonucleotide barcodes (i.e. compatible with next generation sequencing and/or amplification, PCR, digital PCR), or
- affinity tags for example biotin, or high-affinity guest molecules for for example Cucurbit-7-uryl or other Cucurbit-N-uryl host molecules, or FLAG-, Myc-, 3HA, His-tags which are commonly used for affinity purification, or they may be,
- enzyme tags for example horse raddish peroxidas, luciferase
- electron microscopy detectable labels like gold particles
- radioactive tags
- XNAzymes tag are tags that comprise an XNA with catalytic activity
- aptamer tags
- electrical impedance spectroscopy tags

In the sense of the present invention, the detectable label may be fully constituted physically before introduction to the biological sample. It may also be already connected to the affinity reagent before introduction to the biological sample in some cases.

In a particularly preferred embodiment of the present invention, however, the detectable label forms by self-assembly following the introduction of its components into the biological sample, wherein all components may be either simultaneously introduced or introduced in a sequential fashion.

The detectable label may further comprise a backbone, which may be a nucleic acid backbone, for example a flexible oligonucleotide, or a rigid 2D, 3D, structure formed by for example DNA origami or DNA brick technology. Likewise, the backbone may be a dendrimeric structure.

The availability of click-FISH PNA probes (Biomers.net GmbH, Ulm, Germany) or similar click chemistry functionalized PNA oligos allows the particularly cost-efficient generation of fluorescent detectable labels by clicking azide-conjugated dyes to PNA oligos and hybridizing them to for example DNA backbone comprising the label oligonucleotide. Azide-conjugated fluorescent dyes are readily commercially available from for example ATTO-Tec GmbH (Siegen, Germany).

Using the same principle, a detectable label may also be functionalized with a biotin-tag, for affinity purification of said detectable label, which may be of interest, when more complicated detectable labels shall be manufactured. This may be the case for example, when DNA origami-based or DNA brick-technology based backbones are used.

### Affinity reagent conjugation to the first barcode comprising oligonucleotide

In a particularly, preferred embodiment of the present invention resistant barcode oligonucleotides are conjugated via a click chemistry to the affinity reagent. If for example, an antibody shall be barcoded this may be performed by using a site-specific conjugation of the barcode oligonucleotide to IgG with azide-alkyne click chemistry, for which there are commercially available kits for example GlyCLICK Azide Activation kit from Genovis AB (#L1-AZ1-100; Lund, Sweden) which yield a degree of labeling of 2 by site-specific conjugation.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step.

Analogously, aspects described in the context of a method step also represent a description of a corresponding block or item or feature of a corresponding apparatus.

### Reference signs

| | |
|---|---|
| 100a, 100b | Target analyte |
| 102a, 102b, 102c, 300, 308, 400, 1100, 1106, 1110, 1114 | Marker |
| 104a, 104b, 104c | Backbone of affinity reagent |
| 106, 304, 402 | Labelling moiety |
| 110, 1102a, 1102b, 1102c | Barcode oligonucleotide of affinity reagent |
| 112 | Label oligonucleotide of label |
| 116 | Degradation agent |
| 200, 202, 700, 106e, 106f, 106g, 1104, 1108, 1112, 1116 | Label |
| 302 | Long oligonucleotide |
| 306 | Oligonucleotide of labelling moiety |
| 500 | Plurality of affinity reagents |
| 502 | Plurality of labels |
| 702 | Sensitive oligo-dye conjugate |
| 704 | Resistant oligo-dye conjugate |
| 706 | Point modification of label oligonucleotide |
| 902 | Staple strand |
| 1000 | Polystyrene bead |

## Claims

1. A marker (102a, 102b, 102c, 300, 308) for analysing a biological sample comprising:
an affinity reagent with a backbone (104a, 104b, 104c), and a barcode oligonucleotide (110) attached to the backbone (104a, 104b, 104c), wherein the backbone (104a, 104b, 104c) is configured to specifically bind to a target analyte (100a, 100b), and
a label (200, 202) comprising a label oligonucleotide (112) and at least one labelling moiety (106, 304),
wherein the label oligonucleotide (112) and the barcode oligonucleotide (110) of the affinity reagent are configured to hybridise to each other,
wherein one of the label oligonucleotide (112) and the barcode oligonucleotide (110) is sensitive to a degradation agent (116) and the other one of the label oligonucleotide (112) and the barcode oligonucleotide (110) is resistant to the degradation agent (116).

2. The marker according to one of the preceding claims, wherein the backbone (104a, 104b, 104c) of the affinity reagent consists essentially of amino acids.

3. The marker according to claim 2, wherein the backbone (104a, 104b, 104c) of the affinity reagent is an antibody or an antibody fragment.

4. The marker according to claim 1, wherein the backbone (104a, 104b, 104c) of the affinity reagent consists essentially of nucleic acids.

5. The marker according to claim 4, wherein the backbone (104a, 104b, 104c) of the affinity reagent is configured to specifically bind to the target analyte (100a, 100b) by hybridising of a continuous sequence of nucleotides to a complementary continuous target sequence of nucleotides.

6. The marker according to claim 5, wherein the backbone (104a, 104b, 104c) of the affinity reagent has a complex structure and the backbone (104a, 104b, 104c) is configured to specifically bind to the target analyte by its complex structure.

7. The marker according to claim 6, wherein the backbone (104a, 104b, 104c) of the affinity reagent is an aptamer.

8. The marker according to one of the preceding claims, wherein one of the label oligonucleotide (112) and the barcode oligonucleotide (110) consists essentially of a nucleic acid sensitive to a degradation agent (116) and the other one of the label oligonucleotide (112) and the barcode oligonucleotide (110) consists essentially of a nucleic acid analogue resistant to the degradation agent (116).

9. The marker according to claim 8, wherein the label oligonucleotide (112) consists essentially of a natural nucleic acid and the barcode oligonucleotide (110) consists essentially of a nucleic acid analogue.

10. The marker according to claim 9, wherein the nucleic acid analogue comprises at least one of a 1,5-anhydorhexitol nucleic acid, a cyclohexene nucleic acid, a threose nucleic acid, a glycol nucleic acid, a locked nucleic acid, a peptide nucleic acid, a fluoro arabino nucleic acid, a bridged nucleic acid, a morpholino, a phosphorothioate backbone, a L-pentose backbone, a nucleic acid comprising at least one modified nucleoside.

11. The marker according to one of the preceding claims, wherein the at least one labelling moiety (106, 304) is optically detectable.

12. The marker according to one of the preceding claims, wherein the degradation agent (116) is a nuclease.

13. The marker according to one of the preceding claims, wherein the degradation agent (116) and the sensitive label oligonucleotide (112) or sensitive barcode oligonucleotide (110) are configured such that applying the degradation agent (116) releases the barcode oligonucleotide (110) from a label oligonucleotide-barcode oligonucleotide duplex, such that the barcode oligonucleotide (110) is able to hybridise to another label oligonucleotide (112).

14. The marker according to one of the preceding claims, wherein the degradation agent (116) is an artificially engineered peptidase or protease configured to degrade a peptide nucleic acid or a xeno nucleic acid.

15. The marker according to one of the preceding claims, wherein the degradation agent (116) is silver, iodine, or mercury and the sensitive nucleic acid analogue is a phosphorothioate-modified nucleic acid.

16. The marker according to one of the preceding claims, wherein the degradation agent (116) is based on an inorganic or organic chemistry that allows the degradation of the sensitive nucleic acid analogue.

17. A kit comprising at least one marker (102a, 102b, 102c, 300, 308) according to one of the preceding claims and the degradation agent (116).

18. A kit comprising at least one barcode oligonucleotide and chemical components required to conjugate said barcode oligonucleotide to an affinity reagent backbone in order to generate a marker according to one of the claims 1 to 16.

19. A kit comprising at least one label comprising a label oligonucleotide in order to generate a marker according to one of the claims 1 to 16 and the degradation agent.

20. A method for analysing a biological sample comprising at least a target analyte (100a, 100b), the method comprising the steps:
introducing into the biological sample at least a first marker (102a, 102b, 102c, 300, 308) according to one of the preceding claims 1 to 16, comprising an affinity reagent with a backbone (104a, 104b, 104c) specific to the target analyte and a first label (200, 202) with at least a first labelling moiety (106, 304),
generating a first readout of the biological sample with the at least one marker (102a, 102b, 102c, 300, 308),
applying the degradation agent to the biological sample in order to degrade the label oligonucleotide (112) of the marker (102a, 102b, 102c, 300, 308),
introducing at least one second label (200, 202) with at least a second labelling moiety into the biological sample in order to generate a second marker (102a, 102b, 102c, 300, 308), the second label comprising a second label oligonucleotide configured to hybridise to the barcode oligonucleotide of the affinity reagent, and
generating a second readout of the biological sample with the second marker (102a, 102b, 102c, 300, 308).

21. The method according to claim 20, wherein the target analyte is identified based on the first label and the second label associated with the target analyte in the first readout and second readout.

22. The method according to one of the preceding claims 20 or 21, wherein in the step of introducing the at least one first marker, the affinity reagent of the at least one first marker is introduced prior to the respective label of the first marker, and wherein the affinity reagent is immobilised in the biological sample prior to introducing the label of the first marker with the first labelling moiety into the biological sample.

23. The method according to one of the preceding claims 20 to 22, wherein the first labelling moiety and the second labelling moiety have different detectable properties.

24. The method according to one of the preceding claims 20 to 23, wherein after the step of generating the second readout, the steps of applying the degradation agent (116) to degrade the label oligonucleotide (112), adding at least one further label with a further labelling moiety, and generating a further readout of the biological sample, are iteratively repeated.
